# EUROPEAN PATENT SPECIFICATION

(11) **EP 4 332 288 B1**
(45) Date of publication and mention of the grant of the patent: **29.04.2026**
(21) Application number: 21938616.6
(22) Date of filing: 20.05.2021
(51) Int. Cl.: D04H 1/76, A61F 13/15

(54) **RIGID DEMOLDING FORMING DEVICE**
STARRE ENTFORMUNGSFORMUNGSFORMUNGSVORRICHTUNG
DISPOSITIF DE FORMATION DE DÉMOULAGE RIGIDE

(30) Priority: 28.04.2021 CN 202110466675
(43) Date of publication of application: 06.03.2024
(73) Proprietor: Shanghai Zhilian Precision Machinery Co., Ltd., Shanghai 201709 (CN)
(72) Inventor: XIA, Shuangyin, Shanghai 201709 (CN); ZHOU, Guohong, Shanghai 201709 (CN); CHEN, Guangyan, Shanghai 201709 (CN)
(74) Representative: Witte, Weller & Partner Patentanwälte mbB
(86) International application number: PCT/CN2021/094962
(87) International publication number: WO 2022/227145

(56) References cited:
- EP-A1- 3 563 818
- WO-A1-2012/101602
- CN-A- 101 977 578
- CN-A- 103 948 471
- CN-A- 105 813 609
- CN-A- 107 405 222
- CN-A- 107 530 203
- CN-A- 110 446 478
- CN-A- 110 575 313
- CN-A- 110 621 273
- CN-U- 204 766 192
- JP-A- 2014 104 094
- JP-A- 2016 032 554
- JP-A- H0 617 361
- US-A1- 2005 167 874
- US-A1- 2007 246 147

## Description

### FIELD

The present application relates to a rigid demolding forming device. Molding devices are for example known from EP3563818.

### BACKGROUND

In a disposable sanitary product, a core is a main carrier for absorbing liquid, and is generally formed by entanglement and accumulation of dispersed fluff pulp fibers.

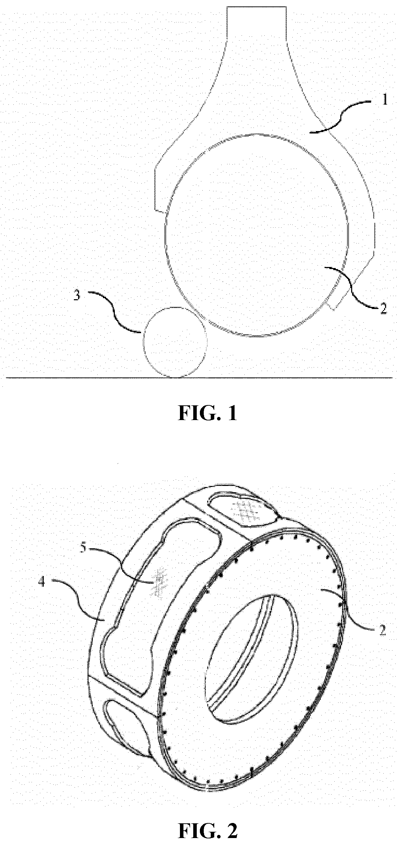

With reference to FIG. 1 and FIG. 2, FIG. 1 and FIG. 2 show an existing apparatus for forming the core of a disposable sanitary product, which includes a forming box 1, a forming cylinder 2 connected to a negative pressure system and a transfer roller 3 for transferring the core from the forming cylinder 2 to a substrate. A mold 4 with a structure corresponding to a shape of the core is fixed on the forming cylinder 2, where the mold 4 includes a forming cavity and a forming fabric 5 arranged at a bottom of the cavity. The forming box 1 fills the forming cavity with absorbent material under an action of wind to form a core with a same shape as the forming cavity. When the forming cavity rotates to a position corresponding to the transfer roller 3, the transfer roller 3 sucks the core from the forming cavity to the transfer roller 3 and transfers it to a conveying line through the transfer roller 3. The problems existing in such structure are as follows.

First, in a process of forming the core, the absorbent material is easy to block fabric holes on the forming fabric, especially in a case that SAP material is also used. The SAP material is easier to block the fabric holes due to its particle shape. However, the forming fabric is fixed at a bottom portion of the forming cavity, so it is impossible to clean the fabric holes during operation, which not only has a great impact on the forming effect of the core, but also reduces the equipment efficiency and increases the energy consumption due to blockage of the fabric holes.

Second, since the formed core needs to be sucked and transferred by the transfer roller, a part of the absorbent material is still hung on the forming fabric during the transfer due to an electrostatic adherence effect or a viscous force effect between the forming fabric and the core, which causes waste of the absorbent material and results in poor forming of the core.

Therefore, there is an urgent need to develop and implement a new alternative solution to solve the above technical problems.

### SUMMARY

In view of this, a rigid demolding forming device is provided according to the present application to solve the above technical problems.

A rigid demolding forming device for forming a core of a disposable absorbent product includes a forming cylinder, a spreading box, a suction box and a forming fabric, where
the forming cylinder includes a rigid bottom plate and a rigid cylinder wall, where the cylinder wall and the bottom plate define an accommodating space, and the cylinder wall is provided with at least one forming hole which penetrates through the cylinder wall along its thickness direction;
the spreading box is arranged in the accommodating space and includes a spreading cavity and a spreading port configured for spreading the absorbent material;
the suction box includes a suction cavity and a suction port, where the suction cavity is connected to a negative pressure device, the suction port and the spreading port are spaced apart from and opposite to each other, and a first area is included between the suction port and the spreading port;
the forming fabric is arranged to separate from the forming cylinder, and a web material is further provided on the forming fabric, where at least a part of the web material and the forming fabric is located in the first area, and the web material is a porous web tissue; and
when the forming cylinder rotates around its axis under an action of external force and carries the forming hole to the first area, the forming hole and the web material form a forming cavity, and the spreading port spreads the absorbent material and fills the absorbent material into the forming cavity to form a core matching a contour of the forming hole.

In an embodiment, the rigid demolding forming device further includes a transfer device for transferring the core to a predetermined position. The transfer device includes a transfer roller. The transfer roller includes an arc-shaped suction area configured for sucking the core onto the transfer roller under a negative pressure of the suction area when the core reaches the suction area and causing the core to move with the transfer roller.

In an embodiment, the forming fabric is wound around the transfer roller.

In an embodiment, at least a part of the cylinder wall is attached to the web material.

In an embodiment, the cylinder wall includes multiple modules which are connected end to end to form a cylinder shape, and one or more forming holes are arranged on each of the multiple modules.

In an embodiment, the spreading box is in a sector shape and the spreading port is in an arc shape.

In an embodiment, the spreading box includes a feeding port. The rigid demolding forming device further includes a feeding cylinder. The feeding cylinder includes a feeding channel, where the feeding port is communicated with the feeding channel. An axis of the feeding cylinder is collinear with an axis of the forming cylinder, and the forming cylinder is configured to rotate around the feeding cylinder.

In an embodiment, the forming fabric is driven by a driving device to continuously pass through the first area.

In an embodiment, the forming fabric is a flexible body, and is connected head-to-tail into a loop.

In an embodiment, the forming fabric is arranged around the suction box.

Beneficial effects: The rigid demolding forming device provided according to the present application includes a forming cylinder, a spreading box, a suction box and a forming fabric, where the forming cylinder is of a rigid structure, the spreading box is arranged in the forming cylinder, the forming fabric is externally arranged, an absorbent material moves from inside to outside, and the forming fabric is arranged to separate from the forming cylinder. On the one hand, the forming fabric can be cleaned conveniently, thereby preventing the fabric hole from being blocked. In addition, the forming fabric and other devices are no longer arranged in the forming hole, such that when the core needs to be detached from the forming hole after the core is formed in the forming hole, product defects or waste of the absorbent material, which are caused by an electrostatic effect or a viscous force effect of devices such as the forming fabric, can be avoided.

### BRIEF DESCRIPTION OF THE DRAWINGS

FIG. 1 is a schematic view showing a device for forming a core of a disposable sanitary product in the conventional technology;
FIG 2 is a schematic perspective view of a demolding cylinder shown in FIG. 1;
FIG. 3 is a schematic view of a rigid demolding forming device according to an embodiment of the present application;
FIG. 4 is a cross-section view taken along line A-A shown in FIG. 3;
FIG. 5 is an enlarged schematic view of area C shown in FIG. 4;
FIG. 6 is an enlarged schematic view of area B shown in FIG. 3; and
FIG. 7 is a schematic perspective view of the rigid demolding forming device shown in FIG .4.

Reference numerals in the drawings are listed as below:
1 forming box; 2, 11 forming cylinder; 3, 16 transfer roller; 4 mold; 5, 14 forming fabric;
111 bottom plate; 112 accommodating space; 113 cylinder wall; 1130 module; 114 forming hole; 115 transmission device;
12 spreading box; 121 spreading cavity; 122 spreading port; 123 feeding port; 124 feeding cylinder; 125 feeding channel;
13 suction box; 131 suction port; 132 first area;
141 fabric hole;
15 core;
17 web material;
18 finishing device.

### DETAILED DESCRIPTION OF THE EMBODIMENTS

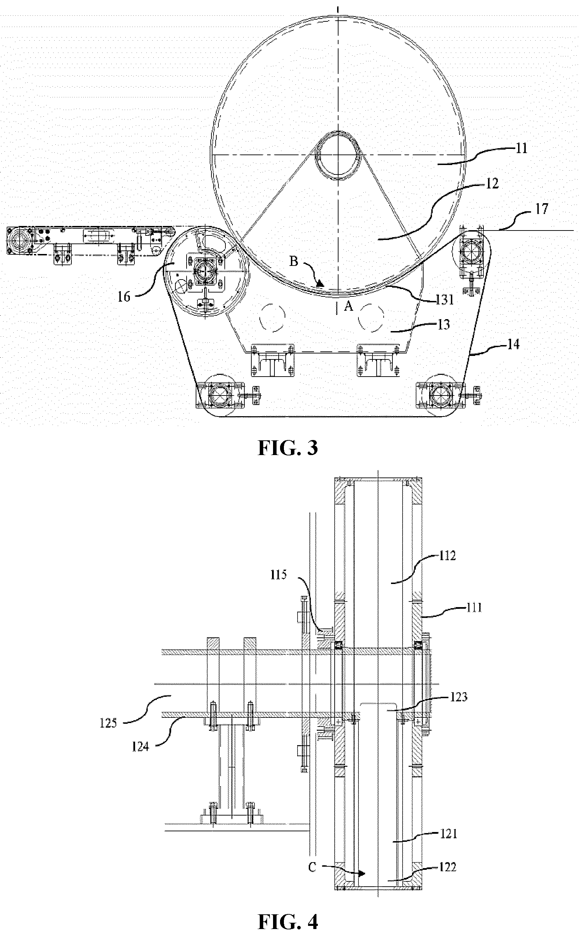

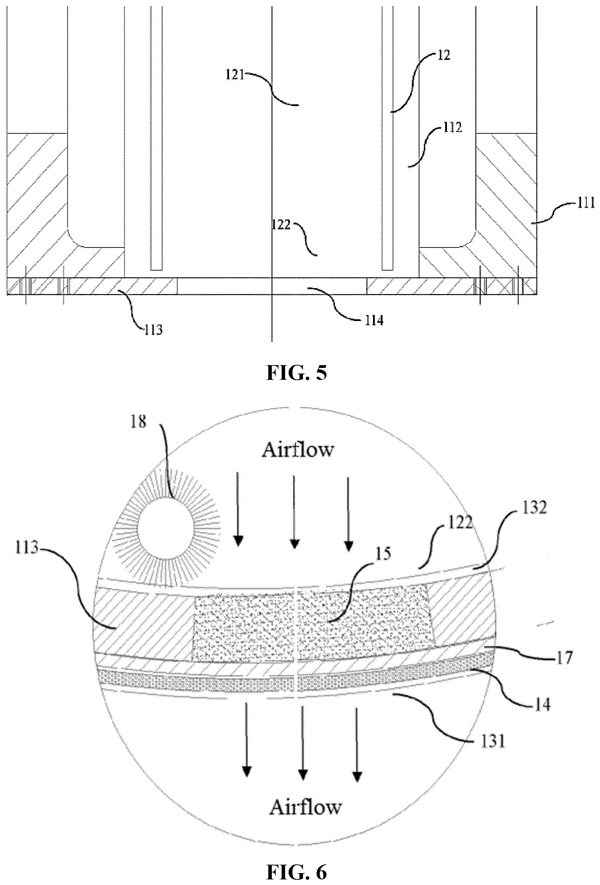

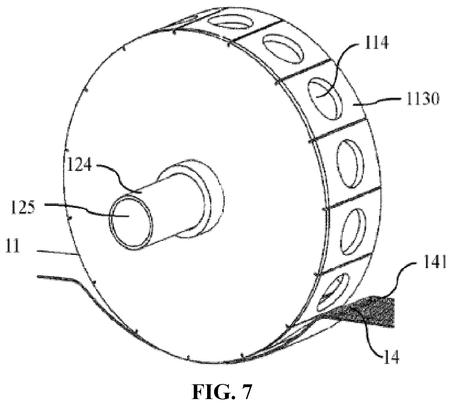

With reference to FIG. 3 to FIG. 7, FIG. 3 is a schematic view showing a rigid demolding forming device, and FIG. 4 to FIG. 7 show structures of the rigid demolding forming device at corresponding positions respectively. The present application will be further described hereinafter with reference to the drawings.

A rigid demolding forming device provided according to the present application is generally applied to a device for forming a core 15, made of an absorbent material, of a disposable sanitary product. The absorbent material may be fiber, such as fluff pulp fiber or man-made fiber or other suitable fiber material, or a mixture of fiber and super absorbent polymer (SAP).

The rigid demolding forming device includes a forming cylinder, a spreading box, a suction box and a forming fabric, and each part of the rigid demolding forming device will be described in detail below.

### 1. Forming cylinder

The forming cylinder 11 is cylindrical, including a pair of bottom plates 111 spaced apart from each other and a cylinder wall 113 connecting the pair of bottom plates 111. The cylinder wall 113 and the pair of bottom plates 111 form an accommodating space 112. The bottom plates 111 and the cylinder wall 113 are rigid bodies. The rigid body means that the forming cylinder is kept in a predetermined shape, such as a cylindrical shape, in a process of forming the core 15, without being deformed in this process.

The cylinder wall 113 is provided with at least one forming hole 114, each of which penetrates through the cylinder wall 113 along its thickness direction. The forming hole 114 has an outline shape and a size matching the shape of the core 15 to be formed. In this embodiment, the core 15 is only shown in a circular shape, and other suitable shapes, such as ellipse, oval, waist, polygon and other regular or irregular shapes, can be set as required, which will not be limited herein. In addition, the meaning of "penetrate through" is that no additional device that will block the airflow will be arranged or included in the forming hole 114.

It can be understood that the cylinder wall 113 may be arranged in a combined form. Specifically, the cylinder wall 113 includes multiple modules 1130, each of which is connected to the bottom plate 111. Each of the modules 1130 is in a tile shape and includes an arc-shaped outer surface and an arc-shaped inner surface. The multiple modules 1130 are connected end to end to form a complete cylinder shape, and each of the modules 1130 may be provided with one or more forming holes 114.

It can be understood that the module 1130 may be detachably connected to the bottom plate 111, so as to facilitate forming the cores 15 with different shapes by replacing different modules 1130.

The forming cylinder 11 rotates along its axis under an action of external force, so that the forming hole 114 arranged on the cylinder wall 113 can move to the first area 132. The first area 132 which will be described in detail in the following description. In an embodiment, the forming cylinder 11 further includes a transmission device 115, through which a power device drives the forming cylinder 11 to rotate, for example.

### 2. Spreading box

The spreading box 12, which is in a sector-shaped box shape, is arranged in the accommodating space 112 of the forming cylinder 11, includes a spreading cavity 121, a feeding port 123 and a spreading port 122, and is configured for spreading the absorbent material through the spreading port 122.

The spreading port 122 is in an arc shape and is arranged to be opposite to a side, facing toward the accommodating space 112, of the cylinder wall 113. When the forming cylinder 11 rotates under the action of external force, the forming hole 114 can pass through an area corresponding to the spreading port 122. In a preferred embodiment, the spreading port 122 has a fixed orientation, toward which the absorbent material is dispersed. It can be understood that the spreading box 12 is fixed in the forming cylinder 11 so that the spreading port 122 has a fixed orientation which is from a center of the forming cylinder 11 to the cylinder wall 113. In this embodiment, the fixed orientation is vertically downward, allowing the spreading port 122 spreading the absorbent material through the spreading port 121 more quickly under the action of airflow and gravity.

It can be understood that a gap between the spreading port 122 and the cylinder wall 113 should be as small as possible to prevent the absorbent material from escaping from the gap between the spreading port 122 and the cylinder wall 113 when the absorbent material is dispersed.

In an embodiment, the rigid demolding forming device further includes a feeding cylinder 124. The feeding cylinder 124 includes a feeding channel 125, and the feeding port 123 is communicated with the feeding channel 125 for conveying the absorbent material into the spreading cavity 121 through the feeding cylinder 124 and spreading the absorbent material through the spreading port 122.

In an embodiment, the feeding cylinder 124 is inserted in the center of the forming cylinder 11, that is, an axis of the feeding cylinder 124 is collinear with an axis of the forming cylinder 11. The forming cylinder 11 is configured to rotate around the feeding cylinder 124, so as to prevent the feeding cylinder 124 from interfering with the rotation of the forming cylinder 11. In this case, the feeding cylinder 124 simultaneously provides support for the spreading box 12 and the forming cylinder 11, that is, the rotation of the feeding cylinder 124 and the spreading box 12 being fixedly arranged in the feeding cylinder 124 can be achieved at the same time.

It can be understood that an air conveying device may be further arranged upstream of the feeding cylinder 124. The air conveying device, on the one hand, is configured for conveying the absorbent material dispersed upstream into the spreading cavity 121, and on the other hand, enabling the spreading cavity 121 to be in a positive pressure state, so that the absorbent material is dispersed through the spreading port 122.

### 3. Suction box

The suction box 13 includes a suction cavity and a suction port 131. The suction port 131 and the spreading port 122 are spaced apart from and opposite to each other, and a first area 132 is included between the suction port 131 and the spreading port 122. It can be understood that the first area 132 is an arc-shaped space between the suction port 131 and the spreading port 122. In an embodiment, the suction port 131 may also be in an arc shape, and an arc surface formed by the suction port 131 and an arc surface formed by the spreading port 122 are spaced apart from and parallel to each other.

It can be understood that when the forming cylinder 11 rotates under the action of external force, the forming hole 114 can move to the first area 132. When the forming cylinder 11 continuously moves, the forming hole 114 can rotate to the first area 132 and then leave the first area 132. When the cylinder wall 113 is provided with multiple forming holes 114, the multiple forming holes 114 can move to the first area 132 in turn, so as to form the corresponding cores 15 in turn.

The suction box 13 is connected to a negative pressure system to form a negative pressure in the suction cavity, so that the airflow in the first area 132 flows from the spreading port 122 to the suction port 131, and in a case that the absorbent material exists in the airflow, the absorbent material will move from the spreading port 122 to the suction port 131 with the airflow.

### 4. Forming fabric

The forming fabric 14 is in a sheet shape and includes multiple fabric holes 141 for supporting a web material 17 arranged on the forming fabric 14. The web material 17 is a porous web tissue to enable the airflow to pass through the web material 17, which may be non-woven fabric, toilet paper and the like and will not be limited herein.

At least a part of the forming fabric 14 and the web material 17 is located in the first area 132. When the forming cylinder 11 rotates around its axis under the action of external force and the forming hole 114 moves to the first area 132, the forming hole 114 and the web material 17 constitute a forming cavity with a surface of the web material 17 as a bottom and an inner wall of the forming hole 114 as a side wall. When the absorbent material is dispersed through the spreading port 122, the absorbent material moves with the airflow from the spreading port 122 to the suction port 131, and when it passes through the first area 132, it is filled into the forming cavity to form the core 15 structure matching the contour shape and size of the forming hole 114. It can be understood that an opening of the forming cavity faces toward the spreading port 122. It can be understood that in this case, the core 15 will be directly formed on the web material 17.

In an embodiment, at least a part of the cylinder wall 113 is attached to the web material 17.

It can be understood that when the web material 17 moves with the forming fabric 14 and the forming fabric 14 moves synchronously with the forming cylinder 11, the forming cylinder 11 continuously forms cores on the web material 17. In this case, the web material 17 simultaneously carries the cores 15 to move in a downstream direction.

In an embodiment, the rigid demolding forming device further includes a web material 17 laying device (not shown) for continuously laying the web material 17 on the forming fabric 14 to continuously form cores on the web material 17.

In an embodiment, the forming fabric 14 can continuously pass through the first area 132 under the drive of a driving device, so that the forming fabric 14 is in a moving state and can convey the formed core 15 to the downstream direction.

In an embodiment, the forming fabric 14 is a flexible body, so that when the forming fabric 14 passes through the first area 132, the web material 17 carried on the forming fabric 14 can be attached to the cylinder wall 113 as much as possible, enabling the gap between the bottom surface and the side wall of the forming cavity to be small, and thus preventing the absorbent material from leaking from the gap between the bottom surface and the side wall, or the forming fabric 14 is attached to the suction port 131, so that the first area 132 has a greater negative pressure effect. Besides, the forming fabric 14 is connected head-to-tail into a loop, and the driving device drives the forming fabric 14 to continuously move. In this case, the forming fabric 14 and the web material 17 continuously form the cores 15 while conveying the cores 15 to the downstream direction.

In an embodiment, the driving device includes a driving wheel and a supporting wheel. The driving wheel and the supporting wheel are arranged around the suction box, and the forming fabric 14 is wound on the driving wheel and the supporting wheel to surround the suction box.

Besides, it can be understood that a moving direction of the forming fabric 14 is the same as that of the forming cylinder 11, and a moving speed is also the same as that of the forming cylinder 11.

In an embodiment, a width of the forming fabric 14 is greater than that of the forming hole 114, and a width of the spreading port 122 is greater than that of the forming hole 114.

In an embodiment, a cleaning device (not shown) may further be arranged on the forming fabric to clean the forming fabric and prevent the fabric holes from being blocked. The cleaning device may be an airflow cleaning device or a brush cleaning device. The airflow cleaning device can clean the fabric holes 141 by spraying airflow on the surface of the forming fabric 14, and the brush cleaning device can clean the fabric holes 141 by brushing the surface of the forming fabric 14. The cleaning device is arranged in a position away from the web material 17.

In an embodiment, the rigid demolding forming device further includes a transfer device for transferring the core 15 to a predetermined position.

In this embodiment, the transfer device includes a transfer roller 16. The transfer roller 16 includes an arc-shaped suction area. When the core 15 moves to the suction area, it is sucked to the transfer roller 16 under the negative pressure of the suction area and moves with the transfer roller 16.

In an embodiment, the forming fabric 14 is wound around the transfer roller 16, so that on the one hand, the movement of the transfer roller 16 is synchronized with the movement of the forming fabric 14, and on the other hand, the transfer roller 16 can directly transfer the core 15.

In an embodiment, the rigid demolding forming device further includes a finishing device 18 for forming cores with generally the same height through finishing. In this embodiment, the finishing device 18 is arranged in the spreading cavity 121 and includes a free end abutting against an inner surface of the cylinder wall 113.

In an embodiment, the finishing device 18 includes a circular section to form a better core surface while reducing wear.

In an embodiment, the finishing device 18 is a cylindrical brush, which includes a cylinder body and multiple bristles fixed on the cylinder body. At least a part of the bristles face towards the suction area and abut against the inner surface of the cylinder wall 113 or reserve only a small gap with the inner surface of the cylinder wall 113. When rotating, the cylindrical brush cleans the surface of the cylinder wall 113, allowing the formed core to have a flat upper surface.

As can be seen from the above description, in the rigid demolding forming device provided according to the present application, the spreading box 12 is arranged in the forming cylinder 11, the forming fabric 14 is arranged externally, the absorbent material moves from the inside to the outside, and the forming fabric 14 is arranged to separate from the forming cavity which is configured for forming the core 15. On the one hand, it is convenient to clean the forming fabric 14, such as providing an airflow cleaning device or a brush cleaning device, so as to prevent the fabric holes 141 from being blocked, thereby improving the efficiency. In addition, the forming fabric and other devices are no longer arranged in the forming hole 114, such that when the core 15 needs to be detached from the forming hole 114 after the core is formed in the forming hole 114, product defects or the waste of absorbent material, which are caused by an electrostatic effect or a viscous force effect of devices such as the forming fabric, can be avoided, thus solving the technical problems that have puzzled those skilled in the art for a long time.

Only preferred embodiments of the present application are described herein with no limitation on the scope of protection of the present application. Any equivalent structure or process from the specification and the drawings of the present application, or direct or indirect application in other related fields, is equally covered by the scope of protection of the present application.

## Claims

1. A rigid demolding forming device for forming a core of a disposable absorbent product, comprising a forming cylinder, a spreading box, a suction box and a forming fabric, wherein
the forming cylinder comprises a rigid bottom plate and a rigid cylinder wall, wherein the cylinder wall and the bottom plate define an accommodating space, and the cylinder wall is provided with at least one forming hole which penetrates through the cylinder wall along its thickness direction;
the spreading box is arranged in the accommodating space and comprises a spreading cavity and a spreading port configured for spreading an absorbent material;
the suction box comprises a suction cavity and a suction port, wherein the suction cavity is connected to a negative pressure device, the suction port and the spreading port are spaced apart from and opposite to each other, and a first area is included between the suction port and the spreading port;
the forming fabric is arranged to separate from the forming cylinder, and a web material is further provided on the forming fabric, wherein at least a part of the web material and the forming fabric is located in the first area, and the web material is a porous web tissue; and
when the forming cylinder rotates around its axis under an action of external force and carries the forming hole to the first area, the forming hole and the web material form a forming cavity, and the spreading port spreads the absorbent material and fills the absorbent material into the forming cavity to form a core matching a contour of the forming hole.

2. The rigid demolding forming device according to claim 1, further comprising a transfer device for transferring the core to a predetermined position, wherein the transfer device comprises a transfer roller, and the transfer roller comprises an arc-shaped suction area configured for sucking the core onto the transfer roller under a negative pressure of the suction area when the core reaches the suction area and causing the core to move with the transfer roller.

3. The rigid demolding forming device according to claim 2, wherein the forming fabric is wound around the transfer roller.

4. The rigid demolding forming device according to claim 1, wherein at least a part of the cylinder wall is attached to the web material.

5. The rigid demolding forming device according to claim 4, wherein the cylinder wall comprises a plurality of modules which are connected end to end to form a cylinder shape, and one or more forming holes are arranged on each of the plurality of modules.

6. The rigid demolding forming device according to claim 2, wherein the spreading box is in a sector shape and the spreading port is in an arc shape.

7. The rigid demolding forming device according to claim 2, wherein the spreading box comprises a feeding port, and the rigid demolding forming device further comprises a feeding cylinder, wherein the feeding cylinder comprises a feeding channel, wherein the feeding port is communicated with the feeding channel, an axis of the feeding cylinder is collinear with an axis of the forming cylinder, and the forming cylinder is configured to rotate around the feeding cylinder.

8. The rigid demolding forming device according to claim 2, wherein the forming fabric is driven by a driving device to continuously pass through the first area.

9. The rigid demolding forming device according to claim 8, wherein the forming fabric is a flexible body, and is connected head-to-tail into a loop.

10. The rigid demolding forming device according to claim 9, wherein the forming fabric is arranged around the suction box.

## Patentansprüche

1. Starre Entformungs-Formvorrichtung zum Formen eines Kerns eines Einweg-Absorptionsprodukts, aufweisend einen Formzylinder, einen Streukasten, einen Saugkasten und ein Formgewebe, wobei
der Formzylinder eine starre Bodenplatte und eine starre Zylinderwand aufweist, wobei die Zylinderwand und die Bodenplatte einen Aufnahmeraum definieren, und die Zylinderwand mit mindestens einem Formloch versehen ist, das die Zylinderwand entlang ihrer Dickenrichtung durchdringt;
der Streukasten in dem Aufnahmeraum angeordnet ist und eine Streukavität und eine Streuöffnung aufweist, die zum Streuen eines Absorptionsmaterials konfiguriert ist;
der Saugkasten eine Saugkavität und eine Saugöffnung aufweist, wobei die Saugkavität mit einer Unterdruckvorrichtung verbunden ist, die Saugöffnung und die Streuöffnung voneinander beabstandet und einander gegenüberliegend sind, und ein erster Bereich zwischen der Saugöffnung und der Streuöffnung enthalten ist;
das Formgewebe so angeordnet ist, dass es von dem Formzylinder getrennt ist, und ein Bahnmaterial ferner auf dem Formgewebe vorgesehen ist, wobei zumindest ein Teil des Bahnmaterials und des Formgewebes in dem ersten Bereich angeordnet ist, und das Bahnmaterial ein poröses Bahngewebe ist; und
wenn der Formzylinder unter einer Einwirkung einer äußeren Kraft um seine Achse rotiert und das Formloch in den ersten Bereich trägt, das Formloch und das Bahnmaterial eine Formkavität bilden, und die Streuöffnung das Absorptionsmaterial streut und das Absorptionsmaterial in die Formkavität füllt, um einen Kern zu bilden, der einer Kontur des Formlochs entspricht.

2. Starre Entformungs-Formvorrichtung nach Anspruch 1, ferner aufweisend eine Transfervorrichtung zum Transferieren des Kerns an eine vorbestimmte Position, wobei die Transfervorrichtung eine Transferwalze aufweist, und die Transferwalze einen bogenförmigen Saugbereich aufweist, der dafür konfiguriert ist, den Kern unter einem Unterdruck des Saugbereichs auf die Transferwalze zu saugen, wenn der Kern den Saugbereich erreicht, und den Kern dazu zu veranlassen, sich mit der Transferwalze zu bewegen.

3. Starre Entformungs-Formvorrichtung nach Anspruch 2, wobei das Formgewebe um die Transferwalze gewickelt ist.

4. Starre Entformungs-Formvorrichtung nach Anspruch 1, wobei zumindest ein Teil der Zylinderwand an dem Bahnmaterial befestigt ist.

5. Starre Entformungs-Formvorrichtung nach Anspruch 4, wobei die Zylinderwand eine Mehrzahl von Modulen aufweist, die Ende an Ende verbunden sind, um eine Zylinderform zu bilden, und ein oder mehrere Formlöcher auf jedem der Mehrzahl von Modulen angeordnet sind.

6. Starre Entformungs-Formvorrichtung nach Anspruch 2, wobei der Streukasten eine Sektorform aufweist und die Streuöffnung eine Bogenform aufweist.

7. Starre Entformungs-Formvorrichtung nach Anspruch 2, wobei der Streukasten eine Zuführöffnung aufweist, und die starre Entformungs-Formvorrichtung ferner einen Zuführzylinder aufweist, wobei der Zuführzylinder einen Zuführkanal aufweist, wobei die Zuführöffnung mit dem Zuführkanal in Verbindung steht, eine Achse des Zuführzylinders mit einer Achse des Formzylinders kollinear ist, und der Formzylinder dafür konfiguriert ist, um den Zuführzylinder herum zu rotieren.

8. Starre Entformungs-Formvorrichtung nach Anspruch 2, wobei das Formgewebe von einer Antriebsvorrichtung angetrieben wird, um kontinuierlich durch den ersten Bereich zu passieren.

9. Starre Entformungs-Formvorrichtung nach Anspruch 8, wobei das Formgewebe ein flexibler Körper ist und Kopf-an-Schwanz zu einer Schleife verbunden ist.

10. Starre Entformungs-Formvorrichtung nach Anspruch 9, wobei das Formgewebe um den Saugkasten herum angeordnet ist.

## Revendications

1. Dispositif de formage à démoulage rigide pour former un noyau d'un produit absorbant jetable, comprenant un cylindre de formage, un caisson de dispersion, un caisson d'aspiration et un tissu de formage, dans lequel
le cylindre de formage comprend une plaque de fond rigide et une paroi de cylindre rigide, dans lequel la paroi de cylindre et la plaque de fond définissent un espace de réception, et la paroi de cylindre est pourvue d'au moins un trou de formage qui pénètre à travers la paroi de cylindre le long de sa direction d'épaisseur ;
le caisson de dispersion est disposé dans l'espace de réception et comprend une cavité de dispersion et un orifice de dispersion configuré pour disperser un matériau absorbant ;
le caisson d'aspiration comprend une cavité d'aspiration et un orifice d'aspiration, dans lequel la cavité d'aspiration est connectée à un dispositif de pression négative, l'orifice d'aspiration et l'orifice de dispersion sont espacés l'un de l'autre et opposés l'un à l'autre, et une première zone est comprise entre l'orifice d'aspiration et l'orifice de dispersion ;
le tissu de formage est agencé pour être séparé du cylindre de formage, et un matériau en bande est en outre prévu sur le tissu de formage, dans lequel au moins une partie du matériau en bande et du tissu de formage est située dans la première zone, et le matériau en bande est un tissu en bande poreux ; et
lorsque le cylindre de formage tourne autour de son axe sous une action d'une force extérieure et porte le trou de formage dans la première zone, le trou de formage et le matériau en bande forment une cavité de formage, et l'orifice de dispersion disperse le matériau absorbant et remplit le matériau absorbant dans la cavité de formage pour former un noyau correspondant à un contour du trou de formage.

2. Dispositif de formage à démoulage rigide selon la revendication 1, comprenant en outre un dispositif de transfert pour transférer le noyau vers une position prédéterminée, dans lequel le dispositif de transfert comprend un rouleau de transfert, et le rouleau de transfert comprend une zone d'aspiration en forme d'arc configurée pour aspirer le noyau sur le rouleau de transfert sous une pression négative de la zone d'aspiration lorsque le noyau atteint la zone d'aspiration et pour amener le noyau à se déplacer avec le rouleau de transfert.

3. Dispositif de formage à démoulage rigide selon la revendication 2, dans lequel le tissu de formage est enroulé autour du rouleau de transfert.

4. Dispositif de formage à démoulage rigide selon la revendication 1, dans lequel au moins une partie de la paroi de cylindre est fixée au matériau en bande.

5. Dispositif de formage à démoulage rigide selon la revendication 4, dans lequel la paroi de cylindre comprend une pluralité de modules qui sont connectés bout à bout pour former une forme de cylindre, et un ou plusieurs trous de formage sont disposés sur chacun de la pluralité de modules.

6. Dispositif de formage à démoulage rigide selon la revendication 2, dans lequel le caisson de dispersion est en forme de secteur et l'orifice de dispersion est en forme d'arc.

7. Dispositif de formage à démoulage rigide selon la revendication 2, dans lequel le caisson de dispersion comprend un orifice d'alimentation, et le dispositif de formage à démoulage rigide comprend en outre un cylindre d'alimentation, dans lequel le cylindre d'alimentation comprend un canal d'alimentation, dans lequel l'orifice d'alimentation est en communication avec le canal d'alimentation, un axe du cylindre d'alimentation est colinéaire avec un axe du cylindre de formage, et le cylindre de formage est configuré pour tourner autour du cylindre d'alimentation.

8. Dispositif de formage à démoulage rigide selon la revendication 2, dans lequel le tissu de formage est entraîné par un dispositif d'entraînement pour passer continuellement à travers la première zone.

9. Dispositif de formage à démoulage rigide selon la revendication 8, dans lequel le tissu de formage est un corps flexible, et est connecté tête-à-queue en une boucle.

10. Dispositif de formage à démoulage rigide selon la revendication 9, dans lequel le tissu de formage est disposé autour du caisson d'aspiration.
